# EUROPÄISCHE PATENTSCHRIFT

(11) **EP 1 606 439 B1**
(45) Veröffentlichungstag und Bekanntmachung des Hinweises auf die Patenterteilung: **17.10.2012**
(21) Anmeldenummer: 04718220.9
(22) Anmeldetag: 08.03.2004
(51) Int. Cl.: D01F 2/06, D01F 2/08, D01D 5/253, A61L 15/28

(54) **MASSIVE REGENERIERTE STANDARDVISKOSEFASER**
SOLID REGENERATED STANDARD VISCOSE FIBRES
FIBRE DE VISCOSE STANDARD REGENEREE SOLIDE

(30) Priorität: 27.03.2003 AT 4882003
(43) Veröffentlichungstag der Anmeldung: 21.12.2005
(73) Patentinhaber: LENZING AKTIENGESELLSCHAFT, 4860 Lenzing (AT)
(72) Erfinder: SCHMIDTBAUER, Josef, A-4840 Vöcklabruck (AT); SCHMIDT, Heinrich, A-4840 Vöcklabruck (AT); BOXAN, Christoph, A-4860 Lenzing (AT); BLAIR, Daniel, Thomas, Morristown, TN 37814 (US)
(74) Vertreter: Nemec, Harald
(86) Internationale Anmeldenummer: PCT/AT2004/000074
(87) Internationale Veröffentlichungsnummer: WO 2004/085720

(56) Entgegenhaltungen:
- EP-A2- 0 716 170
- WO-A-2004/005595
- DE-B- 1 064 678
- NL-C- 102 587
- WALLFISH B ET AL.: "Massgeschneiderte saugfaehige Viskosefasern fuer Hygieneanwendungen" 17. HOFER VLIESSTOFFTAGE, [Online] 7. November 2002 (2002-11-07), Seiten 1-25, XP002259868 LENZING Gefunden im Internet: URL:http://www.hofer-vliesstofftage.de/vor traege/vortrag01.pdf> [gefunden am 2003-10-27]

## Beschreibung

Die vorliegende Erfindung bezieht sich auf eine massive regenerierte Standardviskosefaser und ein Verfahren zur Herstellung dieser Faser.

Fasermaterialien nach dem Stand der Technik, die üblicherweise zur Herstellung von Tampons verwendet werden, sind gewöhnliche Viskosefasern, sogenannte trilobale Viskosefasern und Baumwolle. Das spezifische Absorptionsvermögen dieser Fasern beträgt nach dem untenstehend beschriebenen Syngina-Test ungefähr 4,5 g/g für Baumwolle, 5,5 g/g für gewöhnliche Viskose und 6,5 g/g für trilobale Viskosefasern.

Das Ziel der Tamponhersteller besteht darin, mit einem minimalen Aufwand an Fasermaterial und Kosten einen bestimmten Absorptionsgrad zu erzielen.

Während Baumwolle wegen ihres ungenügenden Absorptionsvermögens als Fasermaterial für Tampons langsam ausgedient hat, sind trilobale Fasern im Vergleich zur gewöhnlichen Viskose viel teurer in der Herstellung und viel schwieriger zu Tampons zu verarbeiten.

Über viele verschiedene Ansätze zur Steigerung des Absorptionsvermögens von Cellulosefasern wurde berichtet:
1. eine chemische Veränderung durch das Aufpfropfen von Monomeren auf die Cellulosefaser
2. eine chemische Veränderung durch den Einbau von absorbierenden Polymeren wie Carboxymethylcellulose, Chitosan, Cellulosecarbamat, Alginat oder Guaran in die Cellulosefasermatrix
3. eine physikalische Veränderung der Fasern, wie z.B. Hohlfasern oder zusammengefallene Hohlfasern, wie beispielsweise aus der US-A 4,129,679 bekannt, oder
4. mehrschenkelige Fasern (sogenannte "trilobale" Fasern), die durch die Verwendung von Spinndüsen mit mehrschenkeligen Extrusionslöchern mit zumindest 3 Schenkeln mit einem Länge-Breite-Verhältnis von 2:1 zu 10:1 erhalten werden, wie beispielsweise aus der EP-A1 0 301 874 bekannt.

Der Nachteil einer chemischen Veränderung der Cellulosefaser besteht darin, dass für sehr empfindliche medizinische Anwendungen wie jener von Tampons ein kostspieliges und zeitaufwendiges toxikologisches und physiologisches Testverfahren nötig ist und das Auftreten des toxischen Schocksyndroms (TSS) die meisten Tamponhersteller von der Verwendung chemisch modifizierter Fasermaterialien abhält, obwohl die Chemikalien als sicher gelten mögen.

Der Nachteil von Hohlfasern und zusammengefallenen Hohlfasern besteht darin, dass sie wegen ihres hohen Wasserrückhaltevermögens schwierig herzustellen sind, aufgrund dessen die Fasern während des Waschens stark anschwellen und wegen der Bildung von Wasserstoffbrücken während des Trocknens aneinanderkleben, was sie im trockenen Zustand brüchig, im nassen Zustand seifig und es schwierig macht, sie aufzubrechen und in ein kardiertes Gewebe zu verarbeiten.

Die Verwendung von trilobalen Fasern erfuhr während der letzten Jahre eine stetige Zunahme, obwohl trilobale Fasern viel schwieriger in ein Tampon zu verarbeiten sind. Die kurzen Schenkel der Fasern sind sehr zerbrechlich und können durch mechanische Kräfte, welche während der Verarbeitung der Fasern, insbesondere während des Kardierens unter Bildung von Faserstaub, ausgeübt werden, leicht beschädigt werden.

Die Herstellung mehrschenkeliger Viskosefasern wurde beispielsweise in den U.S.-Patenten 5,634,914 und 5,458,835 und in der EP-A1 0 301 874 beschrieben. Das dort geoffenbarte Verfahren beschreibt das Spinnen einer üblicherweise verwendeten Viskose, welche eine bestimmte Menge eines im Stand der Technik bekannten Modifikators enthalten kann, durch Extrusionslöcher von mehrschenkeliger Form, insbesondere trilobaler Form, in ein herkömmliches Spinnbad. Das wesentliche Merkmal dieses Verfahrens besteht darin, dass die Form der mehrschenkeligen Extrusionslöcher in der Spinndüse ähnlich der erwünschten Form des Querschnitts der Filamente ist. Gemäß den Lehren dieser Dokumente bestimmt die Geometrie des Spinndüsenlochs die Form des Faserquerschnitts, und durch ein entsprechendes Design der Extrusionslöcher kann ein bestimmtes Länge-Breite-Verhältnis des Faserquerschnitts erhalten werden.

Der Stand der Technik bezüglich mehrschenkeliger Fasern lehrt überdies, dass derartige mehrschenkelige Fasern im Vergleich zu Viskosefasern nach dem Stand der Technik ein gesteigertes Absorptionsvermögen besitzen, und zwar insbesondere in Tampons, und dass solche Fasern zumindest 3 Schenkel haben müssen und dass jeder Schenkel dieser Fasern ein Länge-Breite-Verhältnis von zumindest 2:1, am meisten bevorzugt von 3:1 bis 5:1, aufweisen muss. Je größer das Länge-Breite-Verhältnis ist, desto höher wären der Anteil an freiem Volumen und das Absorptionsvermögen der Fasern, vorausgesetzt, dass die Schenkel nicht so lang und dünn sind, dass sie sich auf sich selbst zurückbiegen.

In diesen Dokumenten ist ohne weitere Belege auch erwähnt, dass unter den Bedingungen eines langsamen Regenerationsspinnens sogar noch höhere Absorptionsvermögen der mehrschenkeligen Fasern erzielt werden können, z.B. durch Absenken des Säurepegels und/oder Erhöhen des Sulfatpegels und/oder Zugabe eines Viskosemodifikators.

Die Tatsache, dass Hohlräume im Querschnitt von Viskosefasern das Absorptionsvermögen dieser Fasern und der daraus hergestellten Produkte erhöhen, ist weiters aus der US-A 4,362,159 bekannt. Zusätzlich offenbart die US-A 4,129,679, dass ein mehrschenkeliger Querschnitt der Viskosefasern aufgrund der Fähigkeit des Filamentbündels, zwischen benachbarten Filamentschenkeln große Mengen an Porenwasser zu enthalten, den aus den Filamenten hergestellten Produkten ein besseres Absorptionsvermögen verleiht.

Überraschenderweise wurde nunmehr herausgefunden, dass es möglich ist, aus Spinndüsen mit mehrschenkeligen Öffnungen eine Viskosefaser zu produzieren, wobei der Querschnitt der Faser eine im wesentlichen dreieckige Form ohne Schenkel mit einem Länge-Breite-Verhältnis von zumindest 2:1 aufweist. Diese Faser vermeidet die Nachteile der mehrschenkeligen Fasern, die Faser ist jedoch überraschenderweise höchst absorptionsfähig, obwohl sie keinen mehrschenkeligen Querschnitt aufweist.

Demgemäß bezieht sich die vorliegende Erfindung auf eine massive regenerierte Standardviskosefaser, welche einen im Wesentlichen dreiecksförmigen Querschnitt hat, dessen Fläche um einen Faktor von weniger als 2,50-fach, vorzugsweise weniger als 2,40-fach, besonders bevorzugt weniger als 2,25-fach, größer ist als die Fläche des größten gleichseitigen Dreiecks, das in diesen Querschnitt eingeschrieben ist, und welche ein nachfolgend definiertes Syngina-Absorptionsvermögen von mehr als 6,0 g/g Faser und einen Titer von 0,5 dtex bis 0,6 dtex aufweist.

Für die Zwecke der vorliegenden Erfindung soll der Begriff "massiv" bedeuten, dass die Faser eine massive, nicht hohle oder zusammengefallene Struktur aufweist.

Der Begriff "Standard" soll bedeuten, dass die Faser eine durch das Viskoseverfahren erhaltene, regenerierte Cellulosefaser ist, welche im konditionierten Zustand eine Reißkraft Bc [cN] von weniger als 1,3 . √T + 2T und im nassen Zustand eine zur Schaffung einer Dehnung von 5% notwendige Kraft Bm [cN] von weniger als 0,5. √T aufweist, wobei T die mittlere lineare Dichte in dtex ist.

Der Querschnitt der erfindungsgemäßen Faser ähnelt annähernd einer Dreiecksform. Diese Dreiecksform kann am besten durch den Vergleich der Fläche des Faserquerschnitts mit der Fläche des größten gleichseitigen Dreiecks, das in diesen Querschnitt eingeschrieben ist, definiert werden.

Je geringer der Unterschied zwischen der Fläche des Faserquerschnitts und der Fläche dieses größten eingeschriebenen Dreiecks ist, desto mehr ähnelt der Faserquerschnitt einer Dreiecksform.

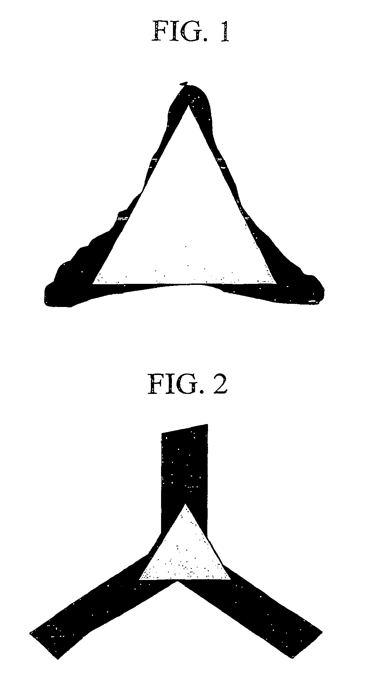

Im Fall eines Querschnitts von annähernder Dreiecksform ist die Fläche des Faserquerschnitts, wie in Fig. 1 ersichtlich, nicht viel größer als die Fläche des größten gleichseitigen Dreiecks, das in diesen Querschnitt eingeschrieben ist.

Gemäß Fig. 2 ist die Fläche des Faserquerschnitts jedoch viel größer als die Fläche des eingeschriebenen Dreiecks, wenn ein Dreieck in eine Faser eingeschrieben ist, die einen trilobalen Querschnitt mit Schenkeln mit einem Länge-Breite-Verhältnis von mehr als 2:1 aufweist.

Gemäß der vorliegenden Erfindung sollte das Verhältnis zwischen der Fläche des Faserquerschnitts und der Fläche des größten eingeschriebenen gleichseitigen Dreiecks weniger als ein Faktor von 2,50, vorzugsweise weniger als 2,40, besonders bevorzugt weniger als 2,25, sein, wobei die Fläche des Querschnitts und dieser Faktor gemäß den untenstehend im Detail dargelegten Verfahren bestimmt werden. Für die Zwecke der vorliegenden Erfindung soll dieser Faktor "Delta-Verhältnis" genannt werden.

Der Artikel "Verzug, Verstreckung und Querschnittsmodifizierung" von Dr. Erich Treiber, Chemiefasern 5/1967, 344-348, offenbart Filamente mit hohem Nassmodul (HWM), die mit einer trilobalen Spinndüse hergestellt wurden. Der Titer des Filaments betrug 3,3 den, die Festigkeit betrug im konditionierten Zustand 4 g/den und im nassen Zustand 2,4 g/den, und die Dehnung betrug 10% bzw. 14%. Der Querschnitt dieses Filaments ist in Fig. 8a) dieser Veröffentlichung dargestellt und stellt ein Delta-Verhältnis von 1,67 dar.

Die Treiber-Veröffentlichung sagt nichts über die Absorptionseigenschaften solcher Filamente. Im Allgemeinen würde der Fachmann erwarten, dass das Wasserrückhaltevermögen von HWM-Fasern beträchtlich geringer ist als jenes von Viskosefasern.

Die erfindungsgemäße Faser liegt vorzugsweise in Form einer Stapelfaser vor.

Der Titer der Faser liegt im Bereich von 0,5 dtex bis 6,0 dtex, vorzugsweise 2,5 dtex bis 4 dtex.

Obwohl die erfindungsgemäße Faser eine massive Faser ist und keinerlei Schenkel mit einem Länge-Breite-Verhältnis von mehr als 2:1 besitzt, wie in der EP-A1 0 301 874 geoffenbart, weist die Faser überlegene Absorptionseigenschaften auf:
ein spezifisches Syngina-Absorptionsvermögen von mehr als 6,0 g/g gemäß dem untenstehend geoffenbarten Testverfahren
ein Wasserrückhaltevermögen, gemessen gemäß DIN 53814 unter Anwendung des Wt-Berechnungsschemas, von 70 bis 110%, vorzugsweise 80 bis 90%.

Überdies ist die erfindungsgemäße Faser aufgrund ihres charakteristischen Querschnitts weniger zerbrechlich als trilobale Fasern und weist eine ausgezeichnete Verarbeitbarkeit während des Kardierens auf.

Die erfindungsgemäße Faser ist für absorbierende Produkte, wie z.B. einen Tampon, perfekt geeignet. Daher stellt die vorliegende Erfindung auch ein absorbierendes Produkt, wie z.B. einen Tampon, bereit, das die erfindungsgemäße Faser in Stapelform enthält.

Es stellte sich heraus, dass die erfindungsgemäße Faser durch ein Verfahren hergestellt werden kann, welches die folgenden Schritte umfasst:
- das Spinnen einer Standardviskosespinnlösung durch eine Spinndüse mit Spinnlöchem in ein Regenerierbad, wodurch Filamente gebildet werden,
- wobei diese Spinnlöcher eine mehrschenkelige Öffnung, vorzugsweise eine dreischenkelige Öffnung, aufweisen,
- wobei die Schenkel dieser Öffnung ein Länge-Breite-Verhältnis von weniger als 3:1 haben
- wobei diese Viskosespinnlösung einen Reifeindex von 10-20° Hottenroth, vorzugsweise 12-16° Hottenroth, aufweist, und
- diese Viskosespinnlösung 0,1-7 Gew.%, vorzugsweise 2-6 Gew.%, Cellulosemodifikator, bezogen auf Cellulose, enthält,
- wobei das Regenerierbad
   - 70 bis 100 g/l, vorzugsweise 75 bis 85 g/l, Schwefelsäure
   - 240 bis 380 g/l, vorzugsweise 270 bis 300 g/l, Natriumsulfat,
   - 5 bis 30 g/l, vorzugsweise 7 bis 12 g/l, Zinksulfat, enthält und
- das Regenerierbad eine Temperatur von 25 bis 55°C, vorzugsweise 30 bis 35°C, hat,
- das Verstrecken und Weiterbehandeln der Filamente gemäß bekannten Verfahren.

Eine Standardviskosespinnlösung ist für die Zwecke der vorliegenden Erfindung eine Viskoselösung nach dem Stand der Technik, welche zur Herstellung von Standardviskosefasern verwendet wird, die typischerweise durch eine Cellulosekonzentration von mehr als 7 Gew.%, vorzugsweise mehr als 8 Gew.%, ein Alkaliverhältnis von weniger als 0,9, typischerweise um 0,6, einen Gamma-Wert unter 50 und einen Reifeindex von 20° Hottenroth und darunter gekennzeichnet sind.

Vorzugsweise ist der Viskosemodifikator ein Polyethylenglycol mit einer Molekülmasse von 600-3000, vorzugsweise 1200-1500.

Alle in der vorliegenden Beschreibung in Gew.% angegebenen Werte werden auf Basis des Cellulosegewichts berechnet.

Bei einer bevorzugten Ausführungsform des Verfahrens werden die Filamente mit einem Fettsäureester behandelt. Vorzugsweise ist der zur Behandlung der Filamente verwendete Fettsäureester ein Polyoxyethylen-Sorbitan-Fettsäureester wie z.B. TWEEN®20 (erhältlich bei ICI Surfactants).

Die Filamente können mit dem Fettsäureester in einer Menge von 0,03 bis 0,7% (w/w auf Cellulosebasis berechnet), vorzugsweise 0,3 bis 0,4%, behandelt werden.

Die Verstreckung und die Weiterbehandlung der Filamente (wie z.B. Schneiden, Ausrüsten und Trocknen) können durch Verfahren bewerkstelligt werden, die dem Fachmann als solche bekannt sind. Typischerweise werden die Filamente nach dem Verlassen des Regenerierbads in einem Zweitbad und/oder in der Luft in einem Streckverhältnis von 40% bis 90%, vorzugsweise 55% bis 70%, verstreckt.

Beim Verfahren nach dem Stand der Technik zur Herstellung von trilobalen Fasern, wie beispielsweise im Versuchsteil der EP-A1 0 301 874 geoffenbart, werden Regenerationsbedingungen angewandt, die für eine rasche Festlegung des Faserquerschnitts in derselben Form wie bei der Extrusion aus dem trilobalen Spinndüsenloch sorgen.

Beim erfindungsgemäßen Verfahren ist es jedoch von entscheidender Bedeutung, dass die Prozessparameter solcherart ausgewogen sind, dass die aus einer trilobalen Spinndüse extrudierten Filamente langsam regeneriert werden, indem ermöglicht wird, dass die Filamente die Schenkel zum Kern hin zusammenziehen, wodurch ein im wesentlichen dreieckiger Querschnitt (im Folgenden als "Δ-förmig" bezeichnet) gebildet wird.

Ein langsames Regenerationsverfahren kann für eine bestimmte Viskosezusammensetzung erzielt werden, indem beispielsweise ein Viskosemodifikator in die Spinnlösung eindosiert wird, und zwar in Kombination mit einer Absenkung der Schwefelsäurekonzentration und einer niedrigen Spinnbadtemperatur.

Es kann nachgewiesen werden, dass zum Erhalt von Δ-förmigen Fasern anstelle von Y-förmigen Fasern beim Spinnen durch Spinndüsen mit trilobalen Öffnungen insbesondere die Kombination eines Modifikators mit einem langsam regenerierenden Spinnbad wesentlich erscheint.

Ohne Zugabe eines Modifikators ist der Querschnitt der aus einer trilobalen Spinndüse erhaltenen Fasern Y-förmig, und zwar sogar dann, wenn die Schwefelsäurekonzentration und die Temperatur des Spinnbads reduziert sind.

Wenn aber eine bestimmte Menge eines Modifikators, z.B. PEG 1500, zur Viskosespinnlösung hinzugefügt wird, ist der Querschnitt der unter denselben Bedingungen gesponnenen Fasern ein Δ-förmiger Querschnitt.

Andererseits weisen die resultierenden Fasern bei Verwendung eines herkömmlichen Spinnbads mit höherer Schwefelsäurekonzentration einen Y-förmigen Querschnitt auf, ungeachtet dessen, ob ein Modifikator eingesetzt wird oder nicht.

Unter Verwendung von Spinndüsen, deren Öffnungen drei Schenkel haben, die ein ziemlich geringes Länge-Breite-Verhältnis, wie z.B. eines, das nur leicht über 2:1 oder unter 2:1 liegt, aufweisen, ist es möglich, sogar dann Δ-förmige Fasern herzustellen, wenn ein Spinnbad mit höherer Schwefelsäurekonzentration eingesetzt wird.

Alternativ können die erfindungsgemäßen Fasern hergestellt werden, indem anstelle von trilobalen Spinndüsen Spinndüsen mit dreieckigen Löchern eingesetzt werden. In diesem Fall müssen die Regenerationsbedingungen in einer Weise angepasst werden, die dazu geeignet ist, den dreieckigen Querschnitt der Faser zu bewahren. Es zeigte sich, dass das Spinnen einer Standardviskosespinnlösung mit einem Alkaliverhältnis von 0,6 mittels einer Spinndüse mit Löchern in Form gleichseitiger Dreiecke in ein herkömmliches Spinnbad oder ein langsam regenerierendes Spinnbad mit oder ohne Zugabe von Polyethylenglycol als Modifikator nicht zu Fasern mit einem Delta-Verhältnis unter 2,50 und einem Syngina-Absorptionsvermögen von mehr als 6 g/g Faser führt. Wird jedoch eine Viskose mit hohem Alkaliverhältnis mittels einer dreieckigen Spinndüse in ein Spinnbad, das eine hohe Konzentration an Zinksulfat enthält, gesponnen, so können Δ-formige Fasern gemäß der vorliegenden Erfindung erhalten werden.

Überraschenderweise wurde herausgefunden, dass Fasern mit dreieckigem Querschnitt, wie obenstehend definiert, ein signifikant besseres Syngina-Absorptionsvermögen als gewöhnliche Viskosefasern aufweisen, und zwar bis zu einem Grad, der gleich oder sogar besser ist als bei trilobalen Viskosefasern, obwohl ihr Querschnitt keinerlei Schenkel mit einem Länge-Breite-Verhältnis von mehr als 2:1 umfasst. Die erfindungsgemäße Faser bietet aufgrund ihrer kompakten Form darüber hinaus signifikante Vorteile bei der Kardierung und Tamponherstellung.

### Kurze Beschreibung der Zeichnungen

Fig. 1 zeigt ein in den Querschnitt einer dreieckigen Faser eingeschriebenes Dreieck.
Fig. 2 zeigt ein in den Querschnitt einer erfindungsgemäßen trilobalen Faser eingeschriebenes Dreieck, (nicht gemäß der Erfindung).
Fig. 3 stellt den zur Durchführung des Syngina-Testverfahrens verwendeten Apparat dar.
Fig. 4 stellt die mechanische Presse dar, die zur Herstellung der Versuchsprobe für das Syngina-Testverfahren verwendet wird.
Fig. 5 ist eine Schnittansicht eines Bestandteils der mechanischen Presse der Fig. 4 gemäß den Linien A-A.
Fig. 6 ist eine Schnittansicht eines weiteren Bestandteils der mechanischen Presse der Fig. 4 gemäß den Linien B-B.
Fig. 7 ist eine vergrößerte Schnittansicht des Bereichs Y in Fig. 5.
Fig. 8 ist eine vergrößerte Schnittansicht des Bereichs Z in Fig. 6.
Fig. 9 bis 15 zeigen die Formen der gemäß den Beispielen 1 bis 7 hergestellten Fasern in vergrößerter Darstellung.

### Testverfahren

### Aufnahme eines Mikrobilds eines Faserquerschnitts

Ein Bündel parallel ausgerichteter Stapelfasern wird durch ein Loch in einer Edelstahlplatte, das einen Durchmesser von 1-2 mm aufweist, gefädelt. Die überstehenden Fasern werden parallel zur Oberfläche der Stahlplatte mit einer Rasierklinge abgeschnitten. Die Platte wird unter ein Mikroskop gelegt, und ein Mikrophoto des Faserquerschnitts wird in einer Vergrößerung von 1070:1 aufgenommen.

### Bestimmung des größten eingeschriebenen gleichseitiges Dreiecks und Bestimmung des Faktors zwischen der Fläche des Faserquerschnitts und der Fläche des größten eingeschriebenen Dreiecks

Diese Bestimmung kann mit einem Personal Computer mittels einer an sich bekannten Grafiksoftware und einer selbst programmierten Rechensoftware wie untenstehend beschrieben durchgeführt werden.
1) Das Profil einer einzelnen Faser wird in eine Bitmap übertragen, wobei Farbwerte die Faser gegen den Hintergrund erkennbar machen. Es werden nur solche Faserprofile für eine grafische Auswertung ausgewählt, die vollständig sichtbar sind und leicht von den benachbarten Profilen isoliert werden können und einen starken Kontrast zum Hintergrund bilden. Der Faserquerschnitt wird mit einem groben Gitter abgedeckt. Die Gittergröße ist ungefähr der zwanzigste Teil der Profilbreite bzw. -höhe.
2) Das größte Dreieck mit dem Schwerpunkt C, das in das Profil gezeichnet werden kann, wird für einen fixierten Winkel zwischen Höhe und y-Achse bestimmt, indem die Seitenlänge des Dreiecks fortlaufend vergrößert wird, bis die Grenze des Profils erreicht ist.
3) Das größte Dreieck wird wie bei 2) für alle Schwerpunkte am Gitter sowie für alle Winkel von 0° bis 360° in Schritten von 0,5° berechnet, und der Schwerpunkt, welcher dem größtmöglichen Dreieck entspricht, wird bestimmt. Danach wird für alle Pixel (x, y) im diesen Punkt umgebenden Bereich das größte Dreieck mit dem Schwerpunkt (x, y) bestimmt, wodurch sich das größtmögliche gleichseitige Dreieck, das eingeschrieben werden kann, ergibt.
4) Die Fläche des Faserquerschnitts wird durch die Farbpixel bestimmt, welche die Faser identifizieren. Die Fläche des optimalen gleichseitigen Dreiecks wird gemäß 3) bestimmt. Das Delta-Verhältnis wird nun erzielt, indem die Fläche des Faserquerschnitts durch die Fläche des optimalen gleichseitigen Dreiecks dividiert wird.

Der Vorgang wird für eine Gesamtanzahl von 12 einzelnen Fasern aus deselben Probe wiederholt, und das durchschnittliche Delta-Verhältnis wird berechnet.

### Syngina-Test:

Der Syngina-Test bewertet das Absorptionsvermögen von Fasern in einem Tampon. Der untenstehend beschriebene Test ist eine vereinfachte Version des EDANA-Testverfahrens ERT 350.0-02.

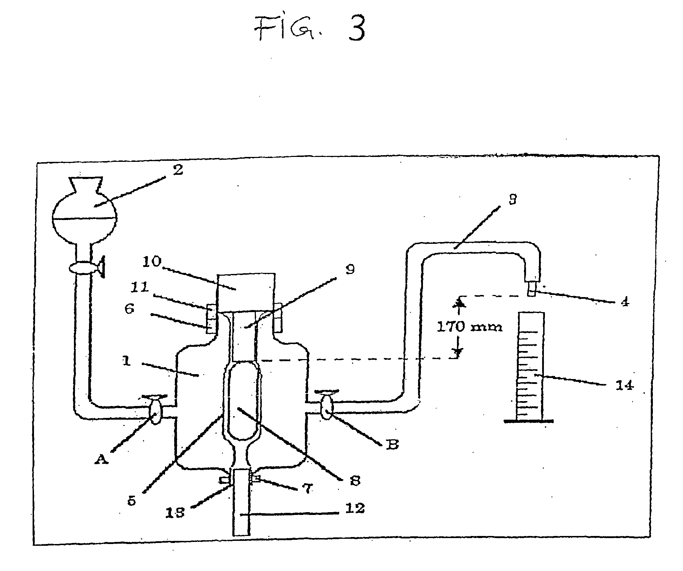

Fig. 3 zeigt den zur Durchführung des Testverfahrens verwendeten Apparat, wobei
- 1: die Messzelle bezeichnet
- 2: ein zuführendes Gefäß bezeichnet
- 3: eine Überlaufleitung bezeichnet
- 4: einen Ablauf bezeichnet
- 5: ein Kondom bezeichnet
- 6,7,11 und 13: jeweils Gummiringe bezeichnen
- 8: einen Tampon oder einen tamponförmigen Stöpsel bezeichnet
- 9: ein Rohr bezeichnet
- 10: ein Füllrohr bezeichnet
- 12: einen Ablauf bezeichnet und
- 14: eine Mensur bezeichnet
- A,B: Ventile bezeichnen.

Das Prinzip des Testverfahrens besteht in der Simulierung der vaginalen Umgebung im Labor durch die Ausübung eines standardmäßigen Drucks auf einen Tampon innerhalb einer elastischen Membran, wobei diese von einem Kondom gebildet wird.

Durch das Einbringen einer bestimmten Menge Flüssigkeit, bis der Tampon undicht wird, können auch das Wasserrückhalte- und Flüssigkeitsabsorptionsvermögen sowie die Wasserverdrängung gemessen werden. Das Tampongewicht wird vor (trocken) und nach dem Test (nass) bestimmt, um das Gewicht der absorbierten Flüssigkeit zu berechnen.

### Reagenzien

Als Syngina-Flüssigkeit wird destilliertes oder entionisiertes Wasser verwendet.

### Herstellung einer Probe

2,75 g Stapelfasern mit einer Feuchtigkeit von 8-11% werden gewogen und in eine mit einem Rotierteil 3 ausgestattete Kardiermaschine vom Typ USTER MDTA 3 gespeist. Die Geschwindigkeit der Kämmwalze beträgt 1390 rpm. Jeder Umlauf dauert 75 s. Das resultierende Kardenband, das etwa 90 cm lang ist, wird dreifach zusammengelegt, um ein Band mit einer Länge von 30 cm zu bilden, das zwischen 2 Walzen gepresst oder auf einer Kalanderwalze verdichtet wird. Die Ausübung eines zu hohen Drucks während des Verdichtens des Kardenbands kann zur Bildung eines steifen, kartonartigen Materials führen, was zu vermeiden ist.

Das Gewicht des verdichteten Kardenbands wird auf 2,70 g eingestellt, und es wird in ein Gerät gegeben, um durch Wicklung einen Zylinder zu formen. Während dieses Vorgangs wird die Rolle von einem 150 g schweren Gegenzylinder gesenkt.

Die Probe wird danach in eine mechanische Presse für Stöpsel gegeben. Dies ist ein mechanisches Gerät, das tamponförmige Stöpsel formen kann. Die Stöpsel haben dasselbe Volumen, dieselbe Masse und dieselbe Faserausrichtung wie ein handelsüblicher Digitaltampon, einschließlich 8 Rillen entlang der Seite des Zylinders. Der Stöpsel wird 10 Minuten lang mit 110 Nm gepresst und unmittelbar vor dem Test aus Redundanzgründen erneut gewogen.

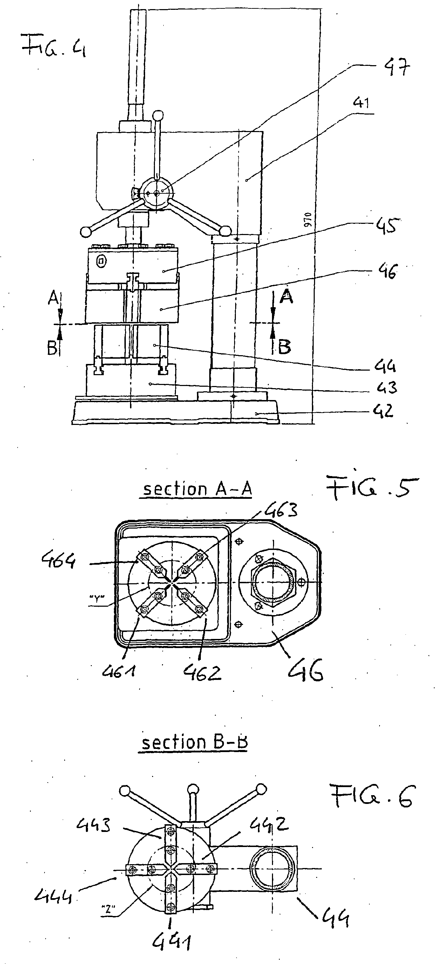

In Fig. 4 bis 8 ist die Presse dargestellt, mittels welcher die tamponförmigen Pressstücke zur Durchführung des Syngina-Tests hergestellt werden.

Die Presse 41 ist auf einer Grundplatte 42 angeordnet und besteht aus einer starr angeordneten unteren Verankerung 43, in welcher eine untere Greifvorrichtung 44 gelagert ist, und einer mittels einer Anhebevorrichtung 47 waagrecht schwenkbaren und senkrecht verfahrbaren oberen Verankerung 45, mit welcher eine obere Greifvorrichtung 46 verbunden ist.

Fig. 5 zeigt einen Schnitt durch die obere Greifvorrichtung 46 gemäß den Linien A-A in Fig. 4. Die obere Greifvorrichtung umfasst vier obere Greifzähne 461-464.

Fig. 6 zeigt einen Schnitt durch die untere Greifvorrichtung 44 gemäß den Linien B-B in Fig. 4. Die untere Greifvorrichtung umfasst vier untere Greifzähne 44.1-444.

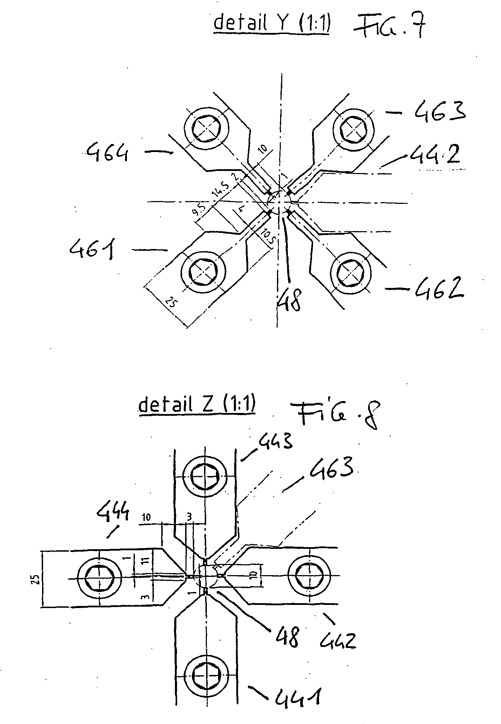

Fig. 7 zeigt einen vergrößerten Ausschnitt des Bereichs Y der Fig. 5. Die genauen Abmessungen der vier oberen Greifzähne 461-464 ergeben sich aus den Bemaßungen in Fig. 7 (in mm). Mit einer strich-punktierten Linie ist ein unterer Greifzahn 442 dargestellt.

Fig. 8 zeigt einen vergrößerten Ausschnitt des Bereichs Z der Fig. 6. Die genauen Abmessungen der vier unteren Greifzähne 441-444 ergeben sich aus den Bemaßungen in Fig. 8 (in mm). Mit einer strich-punktierten Linie ist ein oberer Greifzahn 463 dargestellt.

Zur Herstellung der Probe wird das zuvor gefertigte, verdichtete und zusammengerollte Kardenband (Gewicht = 2,7 g) vertikal in die Öffnung zwischen den unteren Greifzähnen 441-444 eingeführt und mit Hilfe eines Drehmomentschlüssels durch Ausübung eines leichten Drucks auf die unteren Greifzähne fixiert. Anschließend wird die obere Greifvorrichtung 46 eingeschwenkt und heruntergefahren, bis die unteren Greifzähne 441-444 und die oberen Greifzähne 461-464 in einer Ebene liegen und - wie in den Figuren 7 und 8 ersichtlich - jeweils abwechselnd nebeneinander zu liegen kommen. Das zusammengerollte Kardenband befindet sich nun im durch die Greifzähne 441-444 bzw. 461-464 vordefinierten Raum 48 (siehe Figuren 7 und 8) und wird anschließend durch das Zusammenziehen der Greifzähne gepresst. Zu diesem Zweck wird ein Drehmomentschlüssel in einen in der unteren Verankerung 43 vorgesehenen (nicht dargestellten) Vierkantansatz gesteckt und so stark angezogen, dass ein Drehmoment von 110 Nm erreicht wird. Der Pressvorgang dauert 10 Minuten. Auf diese Weise erhält das Pressstück seine charakteristische Form mit 8 Rillen.

Dieser Stöpsel kann ohne weitere Veränderung für den Syngina-Test verwendet werden. Die Länge des Stöpsels beträgt etwa 53 mm, sein Durchmesser beträgt 14-15 mm; für zumindest 7 Tage verändert er seine Längs- oder Radialabmessung nicht.

Wird ein Tampon als Probe verwendet, so muss die Umhüllung oder der Applikator entfernt. werden. Die Testprobe sollte unmittelbar vor dem Test ausgewickelt und das Rückholbändchen abgeschnitten werden.

Die Anzahl der Proben pro Test sollte drei betragen.

### Kondomeinrichtung und -austausch

Ein gerades, nicht eingeschmiertes Kondom mit einer zwischen 17 MPa und 30 MPa liegenden Festigkeit wird als Testmembran verwendet. Das Kondom wird geöffnet und auseinandergezogen. Das Kondom wird in einem Abstand von 20 mm und 160 mm vom offenen Ende markiert.

Das Kondom wird mit Hilfe eines Stabs durch die Kammer 1 des Testapparats (Fig. 3) eingeführt, so dass die 160 mm-Markierung am Rand der kleineren Öffnung der Kammer 1 (dem Boden der Kammer 1) liegt.

Die Spitze des Kondoms wird eingeschnitten und solcherart mit einem Gummiband befestigt, dass die 160 mm-Markierung am Rand der kleineren Öffnung der Kammer 1 bleibt.

Das Kondom wird durch die große Öffnung der Kammer 1 gezogen, so dass die 20 mm-Markierung am Rand der Öffnung liegt, und wird dort mit einem Gummiband befestigt.

Testkondome werden (a), wenn sie undicht sind, (b) - monatlich - ausgetauscht, je nachdem, was zuerst zutrifft.

### Vorgangsweise

Der Tampon oder der gepresste Stöpsel, der gemäß dem obenstehenden Abschnitt "Herstellung einer Probe" hergestellt wurde, wird so genau wie möglich bis auf 0,01 Gramm gewogen. Das Gewicht wird aufgezeichnet.

Während die Kammer 1 des Testapparats leer ist, wird der Tampon 8 in das Kondom 5 gegeben, wie in Fig. 3 gezeigt, so dass die Mitte des Tampons in der Mitte der Kammer 1 ist und das untere Ende (das Ende, wo sich das Rückholbändchen befindet) zum Boden der Kammer 1 positioniert ist. Es ist hilfreich, eine Pinzette zu verwenden, um den Stöpsel in der Mitte dieser Zelle zu platzieren.

Daraufhin wird das Ventil A geöffnet, so dass die Kammer 1 mit Wasser gefüllt wird. Ein kleines Rohr 9 wird in die Kammer 1 eingeführt, so dass es das obere Ende des Tampons /Stöpsels 8 berührt. Das Ventil A wird wieder geschlossen.

Danach wird das Ventil B zum Druckausgleich geöffnet (ein einer Wassersäule von 170 mm entsprechender Druck wird hergestellt, wie in Fig. 3 zu sehen ist). Das Füllrohr 10 wird mit einem Gummiring 11 eingesetzt. 25 ml Testflüssigkeit werden in das Rohr 10 gefüllt. Eine Stoppuhr wird in Gang gesetzt.

Nach 3 Minuten wird das Ventil B geschlossen (außer, es wird über den Ablauf 4 noch immer etwas Wasser verdrängt). Wenn noch Flüssigkeit über das Füllrohr 10 und das kleine Rohr 9 steht, so wird diese mit einer Socorex-Pipette abgesaugt. Das Füllrohr 10 wird entfernt, und die Messzelle wird angehoben.

Das Rohr 9 wird entfernt, das Ventil A wird geöffnet, und das Kondom wird entlastet, wodurch die Herausnahme des Tampons / Stöpsels 8 mit einer Pinzette erleichtert wird. Danach wird das Ventil A geschlossen und die Kammer 1 befestigt.

Der herausgenommene Tampon / Stöpsel wird unverzüglich so genau wie möglich bis auf 0,01 Gramm gewogen. Das Nassgewicht wird aufgezeichnet. Das restliche Wasser wird aus der Kammer 1 abgelassen.

Der Test sollte mit einem neuen Stöpsel von derselben Faserprobe dreimal wiederholt werden.

Für den Test sollte die Kammer 1 ohne Luftblasen gefüllt werden.

### Berechnung und Angabe der Ergebnisse:

Das Absorptionsvermögen jedes Probentampons / Stöpsels wird wie folgt berechnet:
A = B - C, wobei
A = Absorptionsvermögen des Tampons / Stöpsels in Gramm
B = Gewicht in Gramm des gesättigten (nassen) Tampons / Stöpsels
C = Gewicht in Gramm des trockenen Tampons / Stöpsels

Die Ergebnisse werden bis zur ersten Dezimalzahl ausgedrückt. Das durchschnittliche Absorptionsvermögen der gesamten Anzahl von Testproben wird berechnet.

Das spezifische Syngina-Absorptionsvermögen in g Testflüssigkeit/g Faser wird berechnet, indem das durchschnittliche Absorptionsvermögen (A) durch das durchschnittliche Gewicht der trockenen Tampons / Stöpsel (C) in Gramm dividiert wird.

### Wasserrückhaltevermögen

Das Wasserrückhaltevermögen der Fasern wird gemäß dem in DIN 53814 beschriebenen Testverfahren gemessen, wobei das Wt-Berechnungsschema angewandt wird.

### Wasserkapazität

Die Wasserkapazität der Fasern wird gemäß dem Testverfahren hinsichtlich des Absorptionsvermögens von absorbierenden Viskose-Wattierungen, beschrieben in der European Pharmacopoeia 4 01/2002:0034, gemessen.

### Beispiel 1: Δ-förmige Faser

Eine Viskose, enthaltend 8,70% Cellulose, 5,20% Alkali und 2,3% Schwefel, mit einem Reifeindex von 14,2° Hottenroth und einer Kugelfallviskosität von 58 Sekunden (bfs, bestimmt gemäß dem Zellcheming-Merkblatt III/5/E) wurde bei einer Temperatur von 32°C mittels einer trilobalen Spinndüse in ein Regenerierbad, enthaltend 76,5 g/l Schwefelsäure, 272 g/l Natriumsulfat und 10,4 g/l Zinksulfat, gesponnen. Die Spinndüse hatte 625 trilobale Löcher mit 3 Schenkeln von 72x33 µm (Länge-Breite-Verhältnis: 2,18). Vor dem Verspinnen wurden 5 Gew.% einer wässrigen Lösung von Polyethylenglycol 1500 zur Viskose hinzugefügt.

Die Spinngeschwindigkeit betrug 50 m/min. Die Filamente wurden in einem 17 g/l Schwefelsäure enthaltenden, heißen Zweitbad um 55% verstreckt, zu 40 mm langen Stapeln geschnitten, gewaschen, entschwefelt, gebleicht, bei 70°C und einem pH-Wert von 5 mit 10 g/l Polyoxyethylen-Sorbitan-Fettsäureester (Tween 20, erhältlich bei ICI Surfactants) ausgerüstet und getrocknet.

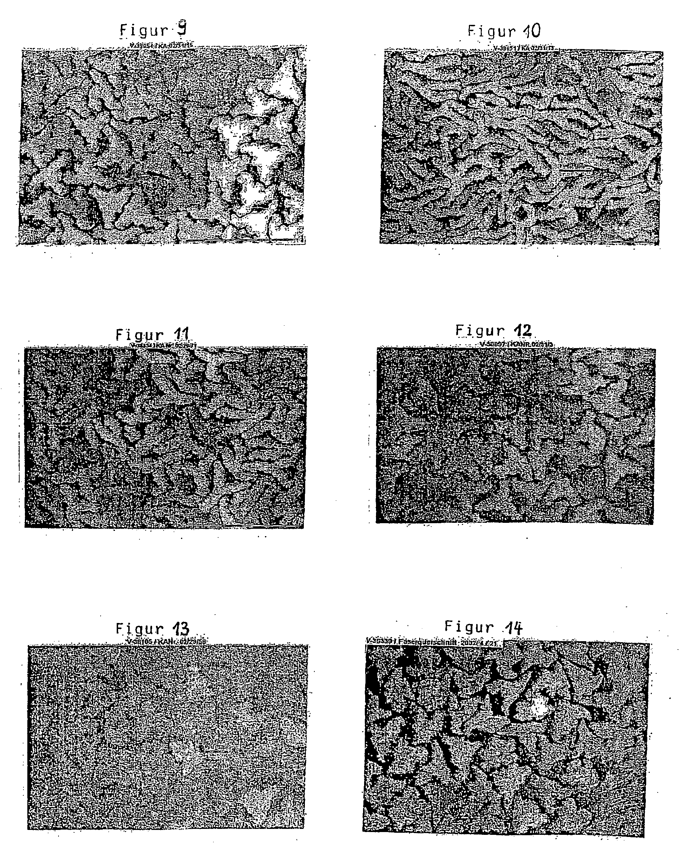

Die Fasern hatten einen Titer von 3,0 dtex, ein Wasserrückhaltevermögen von 103% und einen Wasseraufnahmewert von 23,8 g/g. Das Syngina-Absorptionsvermögen gemäß dem obenstehend beschriebenen Syngina-Test betrug 6,7 g/g. Das Delta-Verhältnis der Faser war 2,26. Ihre typische Form ist in Fig. 9 dargestellt.

### Vergleichsbeispiel 2: Verspinnen ohne Verwendung eines Modifikators

Eine Viskosefaser wurde unter denselben Bedingungen wie in Beispiel 1 beschrieben versponnen, abgesehen davon, dass kein Polyethylenglycol zur Viskose hinzugefügt wurde.

Die Fasern hatten einen Titer von 2,9 dtex, das Wasserrückhaltevermögen betrug 120%, und der Wasseraufnahmewert war 24,8 g/g. Das Syngina-Absorptionsvermögen gemäß dem obenstehend beschriebenen Syngina-Test betrug 5,9 g/g.

Das Delta-Verhältnis der Faser war 4,85. Ihre trilobale Form ist in Fig. 10 dargestellt.

### Vergleichsbeispiel 3: Hoher Schwefelsäuregehalt im Regenerierbad

Eine Viskose, enthaltend 8,65% Cellulose, 5,16% Alkali und 2,3% Schwefel, mit einem Reifeindex von 14° Hottenroth und einer Viskosität von 63 bfs wurde bei einer Temperatur von 49°C mittels einer trilobalen Spinndüse, wie in Beispiel 1 beschrieben, in ein Regenerierbad, enthaltend 131 g/l Schwefelsäure, 367 g/l Natriumsulfat und 11 g/l Zinksulfat, gesponnen. Vor dem Verspinnen wurden 2,5 Gew.% einer wässrigen Lösung von Polyethylenglycol 1500 zur Viskose hinzugefügt.

Die Spinngeschwindigkeit betrug 50 m/min. Die Filamente wurden in einem 19 g/l Schwefelsäure enthaltenden, heißen Zweitbad um 76% verstreckt, zu 40 mm langen Stapeln geschnitten, gewaschen, entschwefelt, gebleicht, bei 70°C und einem pH-Wert von 5 mit 5 g/l Polyoxyethylen-Sorbitan-Fettsäureester (Tween 20, erhältlich bei ICI Surfactants) ausgerüstet und getrocknet.

Die Fasern hatten einen Titer von 3,2 dtex, ein Wasserrückhaltevermögen von 87% und einen Wasseraufnahmewert von 23,7 g/g. Das Syngina-Absorptionsvermögen gemäß dem obenstehend beschriebenen Syngina-Test betrug 6,6 g/g.

Das Delta-Verhältnis der Faser war 4,07. Ihre typische Form ist in Fig. 11 dargestellt.

### Beispiel 4: Δ-förmige Faser

Eine Viskose, enthaltend 8,65% Cellulose, 5,14% Alkali und 2,3% Schwefel, mit einem Reifeindex von 13,6° Hottenroth und einer Viskosität von 65 bfs wurde bei einer Temperatur von 31°C mittels einer trilobalen Spinndüse, wie in Beispiel 1 beschrieben, in ein Regenerierbad, enthaltend 85 g/l Schwefelsäure, 276 g/l Natriumsulfat und 11 g/l Zinksulfat, gesponnen. Vor dem Verspinnen wurden 5 Gew.% einer wässrigen Lösung von Polyethylenglycol 1500 zur Viskose hinzugefügt.

Die Spinngeschwindigkeit betrug 50 m/min. Die Filamente wurden in einem 19 g/l Schwefelsäure enthaltenden, heißen Zweitbad um 55% verstreckt, zu 40 mm langen Stapeln geschnitten, gewaschen, entschwefelt, gebleicht, bei 70°C und einem pH-Wert von 5 mit 10 g/l Polyoxyethylen-Sorbitan-Fettsäureester (Tween 20, erhältlich bei ICI Surfactants) ausgerüstet und getrocknet.

Die Fasern hatten einen Titer von 2,9 dtex. Das Syngina-Absorptionsvermögen gemäß dem obenstehend beschriebenen Syngina-Test betrug 6,9 g/g.

Das Delta-Verhältnis der Faser war 1,91. Ihre typische Form ist in Fig. 12 dargestellt.

### Beispiel 5: Δ-Faser, kurzschenkelige trilobale Spinndüse

Eine Viskose, enthaltend 8,67% Cellulose, 5,15% Alkali und 2,3% Schwefel, mit einem Reifeindex von 15° Hottenroth und einer Viskosität von 62 bfs wurde bei einer Temperatur von 53°C mittels einer trilobalen Spinndüse in ein Regenerierbad, enthaltend 85 g/l Schwefelsäure, 277 g/l Natriumsulfat und 11 g/l Zinksulfat, gesponnen. Die Spinndüse hatte 625 Löcher, wobei jedes Loch auf einem Kern in Form eines gleichseitigen Dreiecks 3 Schenkel von 45x33 µm (Länge-Breite-Verhältnis: 1,36) aufwies. Der Radius des Umkreises beträgt 80 µm. Vor dem Verspinnen wurden 5 Gew.% einer wässrigen Lösung von Polyethylenglycol 1500 zur Viskose hinzugefügt.

Die Spinngeschwindigkeit betrug 50 m/min. Die Filamente wurden in einem 17,6 g/l Schwefelsäure enthaltenden, heißen Zweitbad um 55% verstreckt, zu 40 mm langen Stapeln geschnitten, gewaschen, entschwefelt, gebleicht, bei 70°C und einem pH-Wert von 5 mit 5 g/l Polyoxyethylen-Sorbitan-Fettsäureester (Tween 20, erhältlich bei ICI Surfactants) ausgerüstet und getrocknet.

Die Fasern hatten einen Titer von 3,2 dtex, ein Wasserrückhaltevermögen von 78,5% und einen Wasseraufnahmewert von 18,6 g/g. Das Syngina-Absorptionsvermögen gemäß dem obenstehend beschriebenen Syngina-Test betrug 6,1 g/g.

Das Delta-Verhältnis der Faser war 1,63. Ihre typische Form ist in Fig. 13 dargestellt.

### Beispiel 6: Δ-Faser, dreieckige Spinndüse

Eine Viskose, enthaltend 8,23% Cellulose, 7,15% Alkali und 2,20% Schwefel, mit einem Reifeindex von 14,5° Hottenroth und einer Viskosität von 52 bfs wurde bei einer Temperatur von 49°C mittels einer dreieckigen Spinndüse in ein Regenerierbad, enthaltend 98 g/l Schwefelsäure, 351 g/l Natriumsulfat und 28,2 g/l Zinksulfat, gesponnen. Die Spinndüse hatte 625 Löcher, wobei jedes Loch die Form eines gleichseitigen Dreiecks mit s = 129 µm hatte.

Die Spinngeschwindigkeit betrug 55 m/min. Die Filamente wurden in einem 19,6 g/l Schwefelsäure enthaltenden, heißen Zweitbad um 82% verstreckt, zu 40 mm langen Stapeln geschnitten, gewaschen, entschwefelt, gebleicht, bei 70°C und einem pH-Wert von 5 mit 10 g/l Polyoxyethylen-Sorbitan-Fettsäureester (Tween 20, erhältlich bei ICI Surfactants) ausgerüstet und getrocknet.

Die Fasern hatten einen Titer von 2,96 dtex. Das Syngina-Absorptionsvermögen gemäß dem obenstehend beschriebenen Syngina-Test betrug 6,4 g/g.

Das Delta-Verhältnis der Faser war 2,03. Ihre typische Form ist in Fig. 14 dargestellt.

### Vergleichsbeispiel 7: Trilobale Faser mit 89x25 µm-Spinndüse (Länge-Breite-Verhältnis 3,56)

Eine Viskose, enthaltend 8,80% Cellulose, 5,20% Alkali, mit einem Reifeindex von 13,5° Hottenroth und einer Viskosität von 70 bfs wurde bei einer Temperatur von 30°C mittels einer trilobalen Spinndüse in ein Regenerierbad, enthaltend 76 g/l Schwefelsäure, 266 g/l Natriumsulfat und 10,4 g/l Zinksulfat, gesponnen. Die Spinndüse hatte 625 trilobale Löcher mit 3 Schenkeln von 89x25µm (Länge-Breite-Verhältnis: 3,56). Vor dem Verspinnen wurden 5 Gew.% einer wässrigen Lösung von Polyethylenglycol 1500 zur Viskose hinzugefügt.

Die Spinngeschwindigkeit betrug 50 m/min. Die Filamente wurden in einem 17 g/l Schwefelsäure enthaltenden, heißen Zweitbad um 55% verstreckt, zu 40 mm langen Stapeln geschnitten, gewaschen, entschwefelt, gebleicht, bei 70°C und einem pH-Wert von 5 mit 10 g/l Polyoxyethylen-Sorbitan-Fettsäureester (Tween 20, erhältlich bei ICI Surfactants) ausgerüstet und getrocknet.

Die Fasern hatten einen Titer von 2,97 dtex und einen Wasseraufnahmewert von 25,0 g/g. Das Syngina-Absorptionsvermögen gemäß dem obenstehend beschriebenen Syngina-Test betrug 6,8 g/g.

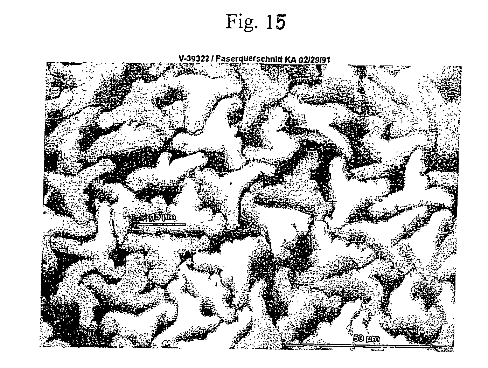

Das Delta-Verhältnis der Faser war 2,64. Ihre typische Form ist in Fig. 15 dargestellt.

## Patentansprüche

1. Massive regenerierte Standardviskosefaser, welche einen im Wesentlichen dreiecksförmigen Querschnitt hat, dessen Fläche um einen Faktor von weniger als 2,50-fach, vorzugsweise weniger als 2,40-fach, besonders bevorzugt weniger als 2,25-fach, größer ist als die Fläche des größten gleichseitigen Dreiecks, das in diesen Querschnitt eingeschrieben ist, welche ein obenstehend definiertes Syngina-Absorptionsvermögen von mehr als 6,0 g/g Faser aufweist und welche einen Titer von 0,5 dtex bis 6,0 dtex aufweist.

2. Faser gemäß Anspruch 1 in Form einer Stapelfaser.

3. Faser gemäß Anspruch 1 oder 2, welche einen Titer von 2,5 dtex bis 4 dtex aufweist.

4. Faser gemäß einem der Ansprüche 1 bis 3, welche ein Wasserrückhaltevermögen, gemessen gemäß DIN 53814, von 70 bis 110%, vorzugsweise 80 bis 90%, aufweist.

5. Verfahren zur Herstellung einer Faser gemäß einem der Ansprüche 1 bis 4, welches die folgenden Schritte umfasst:
- das Spinnen einer Standardviskosespinnlösung durch eine Spinndüse mit Spinnlöchem in ein Regenerierbad, wodurch Filamente gebildet werden,
- wobei diese Spinnlöcher eine mehrschenkelige Öffnung, vorzugsweise eine dreischenkelige Öffnung, aufweisen,
- wobei die Schenkel dieser Öffnung ein Länge-Breite-Verhältnis von weniger als 3:1 haben
- wobei diese Viskosespinnlösung einen Reifeindex von 10-20° Hottenroth, vorzugsweise 12-16° Hottenroth, aufweist, und
- diese Viskosespinnlösung 0,1-7 Gew.%, vorzugsweise 2-6 Gew.%, Viskosemodifikator, bezogen auf Cellulose, enthält,
- wobei das Regenerierbad
- 70 bis 100 g/l, vorzugsweise 75 bis 85 g/l, Schwefelsäure
- 240 bis 380 g/l, vorzugsweise 270 bis 300 g/l, Natriumsulfat,
- 5 bis 30 g/l, vorzugsweise 7 bis 12 g/l, Zinksulfat, enthält und
- das Regenerierbad eine Temperatur von 25 bis 55°C, vorzugsweise 30 bis 35°C, hat,
- das Verstrecken und Weiterbehandeln der Filamente gemäß bekannten Verfahren.

6. Verfahren gemäß Anspruch 5, **dadurch gekennzeichnet, dass** der Viskosemodifikator ein Polyethylenglycol mit einer Molekülmasse von 600-3000, vorzugsweise 1200-1500, ist.

7. Verfahren gemäß Anspruch 5 oder 6, **dadurch gekennzeichnet, dass** die Filamente mit einem Fettsäureester behandelt werden.

8. Verfahren gemäß Anspruch 7, **dadurch gekennzeichnet, dass** der Fettsäureester ein Polyoxyethylen-Sorbitan-Fettsäureester ist.

9. Absorbierendes Produkt, wie z.B. ein Tampon, enthaltend die Faser gemäß einem der Ansprüche 1 bis 4 in Stapelform.

## Claims

1. Solid regenerated standard viscose fibre having an essentially triangle-shaped cross section the area of which is larger than the area of the largest equilateral triangle inscribed into said cross section by a factor of less than 2.50 times, preferably less than 2.40 times, especially preferred less than 2.25 times, having a Syngina absorbency as defined hereinbefore of more than 6.0 g/g fibre and having a titre of from 0.5 dtex to 6.0 dtex.

2. Fibre according to claim 1 in the form of staple fibre.

3. Fibre according to claim 1 or 2, having a titre of from 2.5 dtex to 4 dtex.

4. Fibre according to any one of claims 1 to 3, having a water retention value measured according to DIN 53814 of from 70 to 110%, preferably of from 80 to 90%.

5. Process for the manufacture of a fibre according to any one of claims 1 to 4, comprising the steps of:
- spinning a standard viscose spinning dope through a spinneret comprising spinning holes into a regenerating bath thereby forming filaments,
- said spinning holes having a multi-limbed orifice, preferably a three-limbed orifice
- the limbs of said orifice having an aspect ratio of lower than 3:1
- said viscose spinning dope having a ripening index of 10-20° Hottenroth, preferably 12-16° Hottenroth, and
- said viscose spinning dope containing 0.1-7 wt.%, preferably 2-6 wt.% based on cellulose of a viscose modifier
- said regenerating bath containing
- from 70 to 100 g/l, preferably 75 to 85 g/l sulfuric acid,
- from 240 to 380 g/l, preferably 270 to 300 g/l sodium sulphate,
- from 5 to 30 g/l, preferably 7 to 12 g/l zinc sulphate and
- said regenerating bath having a temperature of from 25 to 55°C, preferably 30 to 35°C,
- stretching and further treating said filaments according to known methods.

6. Process according to claim 5, **characterized in that** the viscose modifier is a polyethylene glycol with a molecular weight of 600-3000, preferably 1200-1500.

7. Process according to claim 5 or 6, **characterised in that** said filaments are treated with a fatty acid ester.

8. Process according to claim 7, **characterized in that** said fatty acid ester is a polyoxyethylene sorbitan fatty acid ester.

9. An absorbent product, such as a tampon, including the fibre according to any one of claims 1 to 4 in staple form.

## Revendications

1. Fibre massive de viscose standard régénérée de section transversale essentiellement triangulaire, dont la surface est plus grande d'un facteur de moins de 2,5 fois, de préférence de moins de 2,4 fois et de façon particulièrement préférable de moins de 2,25 fois que la surface du plus grand triangle équilatéral inscrit dans cette section transversale, et qui présente une capacité d'absorption Syngina, définie plus haut, de plus de 6,0 g/g de fibres, et un titre de 0,5 dtex à 6,0 dtex.

2. Fibre selon la revendication 1, présentant la forme d'une fibre discontinue.

3. Fibre selon les revendications 1 ou 2, dont le titre est compris entre 2,5 dtex et 4 dtex.

4. Fibre selon l'une des revendications 1 à 3, dont la capacité de retenue d'eau mesurée selon DIN 53814 est comprise entre 70 et 110 % et de préférence entre 80 et 90 %.

5. Procédé de fabrication d'une fibre selon l'une des revendications 1 à 4, le procédé comportant les étapes suivantes :
- filage d'une solution standard de filage de viscose à travers une filière de filage dotée de trous de filage dans un bain de régénération, ce qui forme des filaments,
- les trous de filage présentant une ouverture à plusieurs branches, de préférence une ouverture à trois branches,
- les branches de cette ouverture ayant un rapport longueur-largeur inférieur à 3:1,
- la solution de filage de viscose présentant un indice de maturation de 10-20° Hottenroth et de préférence de 12-16° Hottenroth et
- la solution de filage de viscose contenant par rapport à la cellulose de 0,1 à 7 % en poids et de préférence de 2 à 6 % en poids d'agent de modification viscose,
- le bain de régénération contenant :
- de 70 à 100 g/l et de préférence de 75 à 85 g/l d'acide sulfurique
- de 240 à 380 g/l et de préférence de 270 à 300 g/l de sulfate de sodium,
- de 5 à 30 g/l et de préférence de 7 à 12 g/l de sulfate de zinc et
- le bain de régénération ayant une température de 25 à 55°C et de préférence de 30 à 35°C,
- avec ensuite étirage et poursuite du traitement des filaments selon des procédés connus.

6. Procédé selon la revendication 5, **caractérisé en ce que** l'agent de modification viscose est un polyéthylène glycol dont la masse moléculaire est comprise entre 600 et 3 000 et de préférence entre 1 200 et 1 500.

7. Procédé selon les revendications 5 ou 6, **caractérisé en ce que** les filaments sont traités à l'aide d'un ester d'acide gras.

8. Procédé selon la revendication 7, **caractérisé en ce que** l'ester d'acide gras est un ester de polyoxyéthylène-sorbitan-acide gras.

9. Produit absorbant, par exemple tampon, contenant les fibres selon l'une des revendications 1 à 4 sous la forme de fibres discontinues.
